# EUROPEAN PATENT SPECIFICATION

(11) **EP 0 831 881 B1**
(45) Date of publication and mention of the grant of the patent: **12.03.2003**
(21) Application number: 96918201.3
(22) Date of filing: 05.06.1996
(51) Int. Cl.: A61K 38/17

(54) **CETP FOR INCREASING HDL CHOLESTEROL LEVEL**
CETP ZUR ERHÖHUNG DES HDL-CHOLESTEROL-GEHALTS
CETP POUR ACCROITRE LE TAUX DE CHOLESTEROL DES LPHD

(30) Priority: 06.06.1995 US 482454
(43) Date of publication of application: 01.04.1998
(73) Proprietor: Avant Immunotherapeutics, Inc., Needham, Massachusetts 02494 (US)
(72) Inventor: KWOH, Deborah, Y., Carlsbad, CA 92009 (US); BROSTOFF, Steven, W., Carlsbad, CA 92009 (US); CARLO, Dennis, J., Rancho Santa Fe, CA 92067 (US)
(74) Representative: Voelker, Ingeborg Carla Emmy
(86) International application number: PCT/US96/09143
(87) International publication number: WO 96/039168

(56) References cited:
- WO-A-93/11782
- WO-A-95/04755
- DATABASE MEDLINE FILE SERVER STN KARLSRUHE ABSTRACT 95105666, EVANS ET AL: "INHIBITION OF CHOLESTERYL ESTER TRANSFER PROTEIN IN NORMOCHOLESTEROLEMIC AND HYPERCHOLESTEROLEMIC HAMSTERS: EFFECTS ON HDL SUBSPECIES,QUANTITY, AND APOLIPOPROTEIN DISTRIBUTION" XP002014308
- JOURNAL OF BIOLOGICAL CHEMISTRY, vol. 264, no. 24, 25 August 1989, pages 14318-14326, XP000575971 SWENSON T L ET AL: "MECHANISM OF CHOLESTERYL ESTER TRANSFER PROTEIN INHIBITION BY A NEUTRALIZING MONOCLONAL ANTIBODY AND MAPPING OF THE MONOCLONAL ANTIBODY EPITOPE"
- JOURNAL OF BIOLOGICAL CHEMISTRY, vol. 267, no. 25, 5 September 1992, pages 17487-17490, XP000575972 WANG S ET AL: "IDENTIFICATION OF A SEQUENCE WITHIN THE C-TERMINAL 26 AMINO ACIDS OF CHOLESTERYL ESTER TRANSFER PROTEIN RESPONSIBLE FOR BINDING A NEUTRALIZING MONOCLONAL ANTIBODY AND NECESSARY FOR NEUTRAL LIPID TRANSFER ACTIVITY"

## Description

This invention relates generally to the field of immunotherapy and, more specifically, to methods of stimulating an immune response to cholesteryl ester transfer protein (CETP).

### BACKGROUND OF THE INVENTION

Blood cholesterol levels have long been thought to correlate directly with risk of atherosclerotic cardiac disease, the leading cause of heart attacks. More recently, it has been appreciated that blood cholesterol is actually composed of two primary forms: the high density lipoproteins (HDL) and low density lipoproteins (LDL). Rather than being associated with the disease risk, high HDL levels are apparently inversely predictive. In fact, studies have now indicated that HDL has a direct action in protecting against atherosclerosis and may even promote atherosclerosis plaque regression.

Numerous factors are involved in regulating the level of cholesterol in the body. Cholesteryl ester transfer protein (CETP) is an enzyme responsible for transporting cholesterol esters (CE) from HDL to very low density lipoproteins (VLDL) and LDL. VLDL's are eventually converted into LDL. CETP accelerates specifically the exchange of lipid components between pro- and anti-atherogenic lipo protein fractions. In particular, there is a strong inverse correlation between the levels of CETP in the plasma and the levels of HDL cholesterol. CETP activity levels are elevated in individuals suffering from dietary or genetic hypercholesterolemia. Increased levels of CETP activity result in lowered levels of HDL. In contrast, individuals with deficiencies in CETP activity due to mutations in the CETP gene have markedly elevated HDL levels.

The immune systems of higher organisms developed as a means for protecting the individual against invasion by deleterious foreign materials such as viruses, bacteria and parasites. Cells of the immune system are able to distinguish between materials from the individuals own body (termed "self" materials) and foreign material, or antigens. When foreign material enters the body, the immune system mounts a response. Antibodies that specifically recognize and bind to the foreign material are produced (the antibody or humoral response.) In addition, T cells are mobilized to repel the foreign substance (the T cell or cellular response.) Materials which are recognized as self do not normally stimulate such responses except in certain pathological conditions, primarily auto-immune disease. Even where the presence of an endogenous protein is itself deleterious, the immune system cannot serve as a regulator if the material is recognized as self.

Because of HDL'S potentially beneficial effect in preventing atherosclerosis, there exists a need for methods which can be used to increase its level in the serum. Such methods should ideally be specific and reliable and involve as little invasion of the body as possible. The present invention satisfies this need and provides related advantages as well.

### SUMMARY OF THE INVENTION

The present invention provides the use of CETP for preparing a pharmaceutical composition for increasing HDL cholesterol in a mammal by stimulating an immune response that inhibits the function of CETP. Such an immune response can be induced by immunizing with CETP or fragments of CETP (together termed "CETP Peptides") which contain an epitope capable of stimulating such a response. The peptides can be conjugated to a carrier, such as Keyhole Limpet Hemocyanin (KLH) or ovalbumin, in order to increase immunogenicity. Adjuvants can also be administered.

In one embodiment, the fragments of CETP used to raise the antibody response are about ten to twenty amino acids in length and contain sequences homologous to the sequence in rabbit or human CETP.

### BRIEF DESCRIPTION OF THE DRAWINGS

Figure 1 shows the anti-CETP peptide titer during the immunization period for animals immunized with either toxoid conjugated peptide (solid line), free peptide (dashed line) or saline (dotted line). The error bar represents the standard deviation.
Figure 2 shows the CETP activity during the immunization period for animals immunized with either toxoid conjugated peptide (solid line), free peptide (dashed line) or saline (dotted line). The error bar represents the standard deviation.
Figure 3 shows the CETP activity as a function of time after feeding immunized animals a cholesterol diet. The animals were immunized with either toxoid conjugated peptide (solid line) or free peptide (dashed line). The error bar represents the standard deviation.
Figure 4 shows the HDL-cholesterol level as a function of time after feeding immunized animals a cholesterol diet. The animals were immunized with toxoid conjugated peptide (solid line) or free peptide (dashed line). The error bar represents the standard deviation.
Figure 5 shows the LDL-cholesterol level as a function of time after feeding immunized animals a cholesterol diet. The animals were immunized with either toxoid conjugated peptide (solid line) or free peptide (dashed line). The error bar represents the standard deviation.
Figure 6 shows the LDL/HDL-cholesterol ratio as a function of time after feeding immunized animals a cholesterol diet. The animals were immunized with either toxoid conjugated peptide (solid line) or free peptide (dashed line). The error bar represents the standard deviation.

### DETAILED DESCRIPTION OF THE INVENTION

The present invention provides a means to utilize the body's own immune system to lower CETP levels, thereby increasing the level of beneficial HDL cholesterol. The invention provides an effective method of raising HDL in the blood or more specifically, the serum. By utilizing the body's own immune system to increase HDL levels, the invention avoids the problems associated with the repeated administration of drugs, which have undesirable side effects.

According to the present invention, CETP peptide is administered to an appropriate individual in such a manner as to elicit an anti-CETP immune response. The CETP peptide can be chosen to contain an epitope capable of stimulating an antibody or humoral response. Alternatively, the CETP peptide can stimulate a cellular response, or other immune response. CETP peptides can be elected to contain B cell epitopes, sequences capable of stimulating the production of antibodies that specifically recognize and bind to the epitope. Alternatively, CETP peptides can be chosen which stimulate a T cell or more general immune response.

Individuals exhibiting, or at risk of exhibiting, low serum levels of HDL cholesterol are particularly appropriate for such treatment. Serum HDL levels can be determined using methods well-known in the art (See Warnick, G.R. J.Lipid. Res., 19:65 (1978), for example, which is incorporated herein by reference). Serum HDL of less than about 30-35 mg/dl is considered low. Subjects exhibiting a serum HDL level below this level are particularly suitable for the treatment of the invention.

The protein or peptide to be administered can be all or part of the CETP protein, so long as the protein or peptide contains a B cell and/or T cell epitope. As used herein, "CETP peptide" is intended to include both the full length CETP amino acid sequence as well as fragments thereof. The peptides can have a sequence corresponding to or homologous to a mammalian CETP sequence. It will be appreciated that the peptide can differ from the native sequence to some extent so long as it is capable of inducing antibodies that inhibit the activity of CETP.

CETP is a 55 kD protein based on its amino acid sequence, but with post-translational modifications it has an apparent molecular weight of 66-74 kD. The human CETP mRNA sequence is available in Genbank (accession number M30185). The rabbit CETP mRNA sequence is available in Genbank (accession number M27486). The genbank sequences were translated using the MacVector software program (I.B.M., New Haven, Connecticut) to obtain the complete amino acids sequence of human and rabbit CETP.

Because CETP and its peptide derivatives may be recognized as "self" antigens, carriers can be used to increase their immunogenicity. Such carriers are well known in the art and include, for example, such compounds as Keyhole Limpet Hemocyanin (KLH), ovalbumin, Diphtheria toxoid (Wako BioProducts), and Tetanus toxoid (Connaught). The CETP peptides can be conjugated to such carriers by methods well-known in the art. See Current Protocols in Molecular Biology, Ausebell, Brent, Kingston, Moore, Seidman, Smith & Strull eds. (1987), or manufacturers' instructions, which is incorporated herein by reference. The immunogenicity of the peptides can be also increased by administration of a adjuvant. Various adjuvants are well-known and available. See Antibodies: A Laboratory Manual, Harlow and Lane eds., (1988) which is incorporated herein by reference.

The extent of the anti-CETP response induced by the administration of the CETP peptides can be monitored using a variety of assays. For example, competitive format immunoassays can be employed using anti-CETP antibodies or anti-CETP antiserum. Alternatively, the activity level of the CETP in the subject individual can be monitored using, for example a ³H-cholesterol oleate transfer assay. Lasuncion, M.A., et al. Biochem J., 270:441-449 (1990). Reduction in CETP activity is an indirect indication of the anti-CETP response.

The following examples are intended to illustrate but not limit the invention.

### Example 1

### Administration of CETP peptide immunogen

Peptides corresponding to portions of the human, rabbit and rabbit/human CETP were prepared according to standard peptide synthesis protocols. The following peptide sequences were prepared:

H-Cys-Asp-Ser-Gly-Arg-Val-Arg-Thr-Asp-Ala-Pro-Asp-OH
(SEQ ID No.: 1)

H-Cys-Asp-Ala-Gly-Ser-Val-Arg-Thr-Asn-Ala-Pro-Asp-OH
(SEQ ID No.: 2)

H-His-Leu-Leu-Val-Asp-Phe-Leu-Gln-Ser-Leu-Ser-OH.
(SEQ ID No.: 3)

The first peptide (SEQ ID 1) is taken from the Human CETP peptide sequence (residues 131-142 without signal peptide) from Smith and Barakat, Med. Sci. Res., 21:911-912 (1993), which is incorporated herein by reference. The second peptide (SEQ ID 2) is the corresponding rabbit sequence and differs by only 3 amino acids from the human.

The third peptide (SEQ ID 3) is common to both human and rabbit and is an epitope recognized by anti-CETP-monoclonal antibody which is neutralizing. Tall, A.R., J. Lipids Res., 34:1255-1257 (1993).

The peptides were conjugated to ovalbumin by the procedure of Current Protocols in Molecular Biology, supra. Of four New Zealand White rabbits, approximately four months of age, two were injected intramuscularly with 100 micrograms of the ovalbumin-conjugated human peptide (Seq. ID No.: 1) and CFA in PBS and two were injected with the equivalent human/rabbit peptide (Seq. ID No. 3). The animals were boosted twice at one month intervals with the same peptides in incomplete Freund's adjuvant (IFA).

### Example 2

### Immunization with toxoid conjugated CETP

Three groups of female New Zealand rabbits (3 animals per group) were immunized with either toxoid conjugated peptide, free peptide or saline. The immunizations were administered once every seven weeks for a total of four immunizations during the immunization period. Each immunization was intradermally administered at ten sites on the back of each animal. Each immunogen was emulsified in IFA and each site on each animal received 100 µg of emulsion or 1 ml per animal per immunization.

The peptide used for the toxoid conjugated peptide and free peptide was the same, H-His-Leu-Leu-Val-Asp-Phe-Leu-Gln-Ser-Leu-Ser-OH (SEQ ID No.: 3). The peptide of the toxoid conjugated peptide was conjugated to either diphtheria-toxoid or tetanus-toxoid. Both toxoid conjugated peptides were administered to the animals in the toxoid conjugated peptide group. The diphtheria-toxoid conjugated peptide and tetanus-toxoid conjugated peptide were administered in alternative immunizations to avoid carrier-induced immunosuppression. (See Gaur, A., Int. Immunol., 2:151 (1990), for example, which is incorporated herein by reference).

Before, during and for eight weeks after the immunization period, all animals were fed normal chow. Eight weeks after the last immunization, animals in the toxoid conjugated peptide and free peptide groups were switched to and remained on a feed containing 1% cholesterol. All animals had free access to food and water during the experiment except for a fourteen hour fast prior to blood sampling.

Anti-CETP peptide titer was determined by an ELISA assay. Briefly, peptides conjugated to KLH were coated on microtiter plates, the plates incubated with diluted serum and bound antibody detected with a goat-α-rabbit-HRP antibody. The anti-CETP peptide titer was reported as the reciprocal of the dilution that gave 50% of the maximal response.

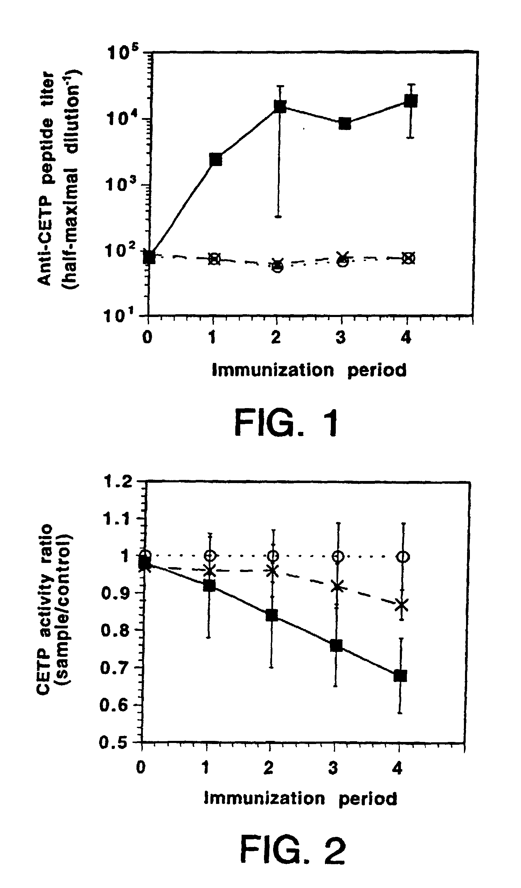

A significant anti-CETP peptide titer (greater than 10³) was produced in the toxoid conjugated peptide group during the immunization period. In contrast, the animals in the free peptide and saline groups did not produce a significant anti-CETP peptide titer. The anti-CETP peptide titer resulting during the immunization period is summarized in Figure 1.

CETP activity was determined prior to the first immunization (the "pre-bleed period") and until the experiment was terminated. CETP activity was determined by isolating HDL from human serum and labeled ³H-cholesterol oleate using well known methods (See, Lasuncion, M.A., Biochem. J., 270:441 (1990), for example, which is incorporated herein by reference). The ³H-HDL (3000 cpm) was incubated for one hour at 37°C with VLDL and 2.5 µl sample serum in a total volume of 50 µl of 50 mM Tris, 150 mM NaCl, 2 mM EDTA at pH 7.4. The reaction was stopped with 450 µl human serum and precipitated by phosphotungstic acid and MgCl₂. The supernatant and resuspended precipitate were counted in a scintillation counter and the results reported as a CETP activity ratio which is the percentage of the ³H-cholesterol ester transferred normalized to the average percent transfer of the saline group.

A significant decrease in the CETP activity was found for animals in the toxoid conjugated group. The CETP activity during the immunization period is summarized in Figure 2.

Purified antibody from animals in the toxoid conjugated peptide group inhibited CETP activity by 42%. In comparison, purified antibody from animals in the saline group inhibited CETP activity by less than 2%. The monoclonal antibody TP-2, known to react with CETP, inhibited CETP activity by 52% (See Whitlock, M.E., J. Clin. Invest., 84:129 (1989), for example). The antibodies to CETP produced by the animals in the toxoid conjugated peptide group thus result in the decreased CETP activity that occurs in that group. Antibodies were purified from serum using well known HPLC high capacity protein-A column chromatography methods.

Total cholesterol, HDL-cholesterol, LDL-cholesterol and triglyceride levels were determined during the pre-bleed period, the immunization period and after immunization. The LDL cholesterol was estimated according to the Friedewald, Ley and Fredrickson equation (See Friedewald, W.T., Clin Chem., 18:499 (1972), herein incorporated by reference).

During the pre-bleed period and while the animals were fed normal chow, no significant difference was detected in cholesterol or triglyceride levels among groups. The total cholesterol, HDL-cholesterol, LDL-cholesterol and triglyceride levels during these periods are summarized in Table 1.

**Table 1.**

| Comparison of serum cholesterol and triglyceride levels in rabbits for each immunization period. | | | | | |
|---|---|---|---|---|---|
| Immunization | Period | Total Cholesterol (mg/dL)* | HDL Cholesterol (mg/dL)* | LDL Cholesterol (mg/dL)* | Total Triglycerides (mg/dL)* |
| Saline | 0 (Pre-bleed) | 52.8 ± 8.0 | 22.9 ± 2.1 | 21.3 ± 6.0 | 43.0 ± 3.3 |
| | 1 (Weeks 0-6) | 53.9 ± 8.6 | 28.1 ± 3.3 | 17.6 ± 7.6 | 35.9 ± 0.8 |
| | 2 (Weeks 7-14) | 54.7 ± 5.0 | 30.9 ± 2.7 | 16.2 ± 2.9 | 41.3 ± 2.6 |
| | 3 (Weeks 15-21) | 40.9 ± 6.6 | 25.3 ± 4.2 | 7.7 ± 3.0 | 39.4 ± 1.1 |
| | 4 (Weeks 22-28) | 40.5 ± 3.5 | 25.4 ± 3.4 | 7.9 ± 1.0 | 36.2 ± 4.1 |
| | | | | | |
| Free peptide | 0 (Pre-bleed) | 46.2 ± 0.9 | 20.2 ± 1.9 | 16.3 ± 4.1 | 48.5 ± 9.2 |
| | 1 (Weeks 0-6) | 46.3 ± 0.5 | 25.3 ± 1.7 | 13.3 ± 2.8 | 38.9 ± 9.3 |
| | 2 (Weeks 7-14) | 53.7 ± 6.4 | 29.3 ± 1.1 | 12.6 ± 6.3 | 49.2 ± 10.0 |
| | 3 (Weeks 15.21) | 39.7 ± 3.7 | 24.6 ± 1.3 | 6.5 ± 5.3 | 43.3 ± 16.6 |
| | 4 (Weeks 22-28) | 38.5 ± 3.5 | 23.8 ± 1.4 | 6.3 ± 4.4 | 42.3 ± 15.0 |
| | | | | | |
| Conjugated peptide | 0 (Pre-bleed) | 57.8 ± 28.0 | 23.7 ± 11.2 | 24.1 ± 13.8 | 50.3 ± 14.9 |
| | 1 (Weeks 0-6) | 56.9 ± 15.8 | 30.9 ± 11.3 | 18.1 ± 7.7 | 46.2 ± 4.3 |
| | 2 (Weeks 7-14) | 44.0 ± 15.0 | 26.7 ± 8.6 | 10.3 ± 4.5 | 34.9 ± 9.6 |
| | 3 (Weeks 15-21) | 44.0 ± 12.5 | 27.0 ± 8.5 | 10.4 ± 2.5 | 33.2 ± 11.1 |
| | 4 (Weeks 22-28) | 48.0 ± 10.4 | 28.0 ± 6.5 | 12.5 ± 4.6 | 38.0 ± 9.9 |

| | | | | | |
|---|---|---|---|---|---|
| * Each value represents the mean±standard deviation for 6 bleeds (pre-bleed period) or 7 bleeds for subsequent periods. | | | | | |

When animals were switched to a 1% cholesterol diet, the total cholesterol, the LDL-cholesterol and CETP activity significantly increased relative to that on the normal chow diet for animals in both the toxoid conjugated peptide group and free peptide group. The total cholesterol, HDL-cholesterol, LDL-cholesterol, total triglycerides, HDL-triglycerides, CETP activity and anti-CETP peptide titer before and after cholesterol feeding are summarized in Table 2.

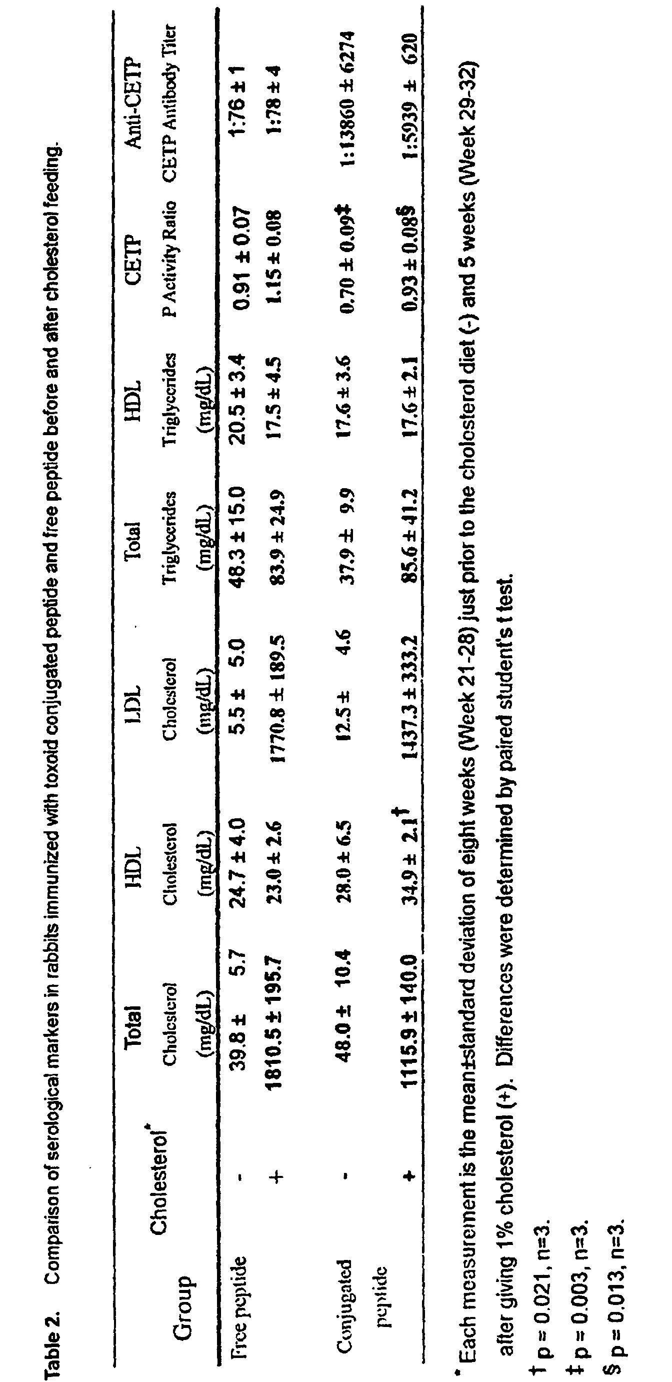

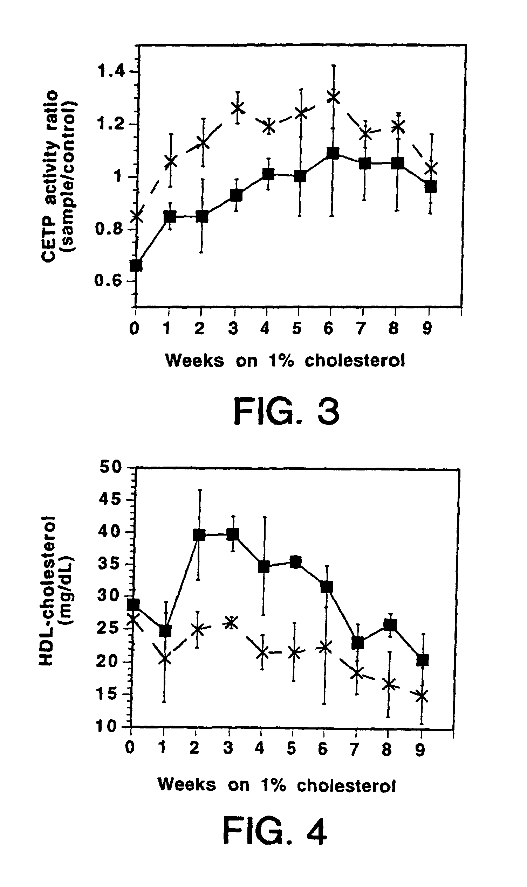

The CETP activity for animals on a cholesterol diet was significantly lower for the toxoid conjugated peptide group relative to that of the free peptide group. The CETP activity for such animals as a function of time on a cholesterol diet is summarized in Figure 3.

The HDL-cholesterol level for animals on a cholesterol chow diet was significantly higher for the toxoid conjugated peptide group relative to the free peptide group. The HDL-cholesterol level for such animals as a function of time on a cholesterol diet is summarized in Figure 4.

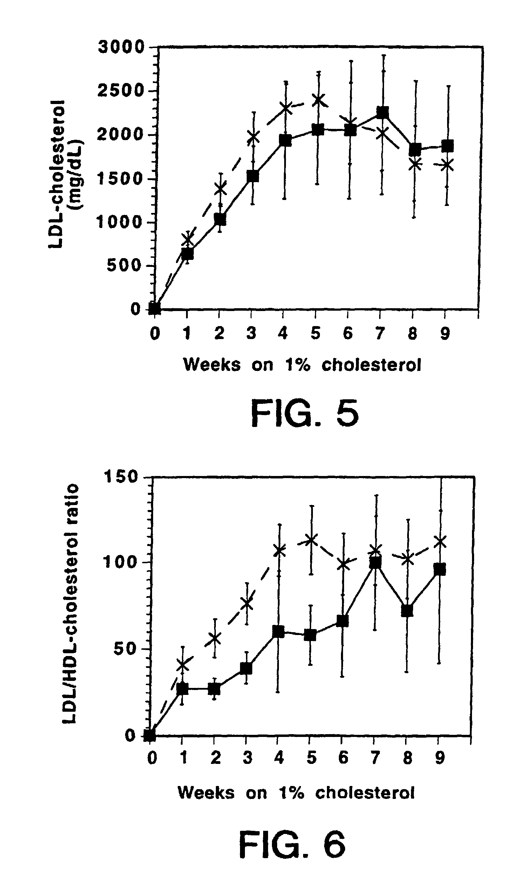

The LDL-cholesterol level for animals on a cholesterol diet was not significantly different for the toxoid conjugated peptide group relative to the free peptide group. The LDL-cholesterol level for such animals as a function of time on a cholesterol diet is summarized in Figure 5.

The LDL/HDL-cholesterol level ratio for animals on a cholesterol chow diet was significantly higher for the free peptide group relative to the toxoid conjugated peptide group. The LDL/HDL-cholesterol level ratio for such animals as a function of time on a cholesterol diet is summarized in Figure 6.

The results show that immunizing animals with the toxoid conjugated peptide raises the HDL-cholesterol level in animals fed a cholesterol diet. Low HDL-cholesterol levels are associated with increased risk of atherosclerosis regardless of the LDL-cholesterol level. Immunization against CETP thus can reduce the risk of atherosclerosis by raising the HDL-cholesterol level.

Although the invention has been described with reference to the presently preferred embodiments, it should be understood that various modifications can be made without departing from the spirit of the invention. Accordingly, the invention is limited only by the following claims.

## Claims

1. The use of full-length cholesteryl ester transfer protein (CETP) for preparing a pharmaceutical composition for increasing high density lipoprotein (HDL) cholesterol in a mammal exhibiting or at risk of exhibiting low levels of serum HDL.

2. The use of full-length cholesteryl ester transfer protein (CETP) for preparing a pharmaceutical composition for reducing the risk of atherosclerosis in a mammal exhibiting or at risk of exhibiting low levels of high density lipoprotein (HDL).

3. The use of claim 1 or 2, wherein said full-length CETP is substantially purified CETP.

4. The use of claims 1 to 3, wherein said full-length CETP is human CETP.

5. The use of claims 1 to 3, wherein said full-length CETP is rabbit CETP.

6. The use of claims 1 to 3, wherein said full-length CETP is the native CETP of said mammal.

7. The use of claims 1 to 3, wherein said full-length CETP differs from the native CETP of said mammal.

8. The use of claims 1 to 7, wherein said mammal is a human.

9. The use of claims 1 to 8, further comprising the use of a carrier.

10. The use of claim 9, wherein said carrier is selected from the group consisting of keyhole limpet hemocyanin (KLH), ovalbumin, diphtheria toxoid, and tetanus toxoid.

11. The use of claims 1 to 10, further comprising the use of an adjuvant.

12. The use of claims 1 to 11, wherein said pharmaceutical composition is for repeated administration.

13. The use of claims 2 to 12, wherein said atherosclerosis is atherosclerotic heart disease.

## Patentansprüche

1. Verwendung eines Volllängen-Cholesterylester-Transferproteins (CETP) zur Herstellung einer pharmazeutischen Zusammensetzung zur Erhöhung des Cholesterins der Lipoproteine mit hoher Dichte (high density lipoprotein; HDL) in einem Säuger, der niedrige Anteile an Serum-HDL aufweist oder für das Aufweisen niedriger Anteile an Serum-HDL ein Risiko trägt.

2. Verwendung des Volllängen-Cholesterylester-Transferproteins (CETP) zur Herstellung einer pharmazeutischen Zusammensetzung zur Verminderung des Risikos für Arteriosklerose in einem Säuger, der niedrige Anteile an Lipoprotein mit hoher Dichte (high density lipoprotein; HDL) aufweist oder für das Aufweisen niedriger Anteile an Lipoprotein mit hoher Dichte (high density lipoprotein; HDL) ein Risiko trägt.

3. Verwendung nach Anspruch 1 oder 2, bei der das Volllängen-CETP im wesentlichen gereinigtes CETP ist.

4. Verwendung nach den Ansprüchen 1 bis 3, bei der das Volllängen-CETP humanes CETP ist.

5. Verwendung nach den Ansprüchen 1 bis 3, bei der das Volllängen-CETP Kaninchen-CETP ist.

6. Verwendung nach den Ansprüchen 1 bis 3, bei der das Volllängen-CETP das native CETP des Säugers ist.

7. Verwendung nach den Ansprüchen 1 bis 3, bei der sich das Volllängen-CETP von dem nativen CETP des Säugers unterscheidet.

8. Verwendung nach den Ansprüchen 1 bis 7, bei der der Säuger ein Mensch ist.

9. Verwendung nach den Ansprüchen 1 bis 8, die ferner die Verwendung eines Trägers umfaßt.

10. Verwendung nach Anspruch 9, bei der der Träger aus der Gruppe bestehend aus Hämocyanin der Schlüsselloch-Napfschnecke (keyhole limpet hemocyanin; KLH), Ovalbumin, Diphtherie-Toxoid und Tetanus-Toxoid ausgewählt ist.

11. Verwendung nach den Ansprüchen 1 bis 10, die ferner die Verwendung eines Adjuvans umfaßt.

12. Verwendung nach den Ansprüchen 1 bis 11, bei der die pharmazeutische Zusammensetzung zur wiederholten Verabreichung dient.

13. Verwendung nach den Ansprüchen 2 bis 12, bei der die Arteriosklerose eine arteriosklerotische Herzerkrankung ist.

## Revendications

1. Utilisation de la protéine de transfert des esters de cholestérol (CETP) de taille complète pour préparer une composition pharmaceutique pour augmenter le cholestérol des lipoprotéines de haute densité (HDL) chez un mammifère présentant ou à risque de présenter de faibles niveaux de HDL sériques.

2. Utilisation de la protéine de transfert des esters de cholestérol (CETP) de taille complète pour préparer une composition pharmaceutique pour diminuer le risque d'athérosclérose chez un mammifère présentant ou à risque de présenter de faibles niveaux de lipoprotéines haute densité (HDL).

3. Utilisation selon la revendication 1 ou 2, dans laquelle ladite CETP de taille complète est une CETP essentiellement purifiée.

4. Utilisation selon les revendications 1 à 3, dans laquelle ladite CETP de taille complète est la CETP humaine.

5. Utilisation selon les revendications 1 à 3, dans laquelle ladite CETP de taille complète est la CETP de lapin.

6. Utilisation selon les revendications 1 à 3, dans laquelle ladite CETP de taille complète est la CETP native dudit mammifère.

7. Utilisation selon les revendications 1 à 3, dans laquelle ladite CETP de taille complète diffère de la CETP native dudit mammifère.

8. Utilisation selon les revendications 1 à 7, dans laquelle ledit mammifère est un humain.

9. Utilisation selon les revendications 1 à 8, comprenant en outre l'utilisation d'un vecteur.

10. Utilisation selon la revendication 9, dans laquelle ledit vecteur est choisi dans le groupe comprenant l'hémocyanine de patelle (KLH), l'ovalbumine, l'anatoxine diphtérique, et l'anatoxine tétanique.

11. Utilisation selon les revendications 1 à 10, comprenant en outre l'utilisation d'un adjuvant.

12. Utilisation selon les revendications 1 à 11, dans laquelle ladite composition pharmaceutique est destinée à une administration répétée.

13. Utilisation selon les revendications 2 à 12, dans laquelle ladite athérosclérose est une athérosclérose coronarienne.
